# EUROPEAN PATENT SPECIFICATION

(11) **EP 2 327 360 B1**
(45) Date of publication and mention of the grant of the patent: **31.10.2012**
(21) Application number: 10192866.1
(22) Date of filing: 29.11.2010
(51) Int. Cl.: A61B 5/04, A61B 5/0428, G06F 17/00

(54) **Method and apparatus for improving signal to noise ratio of ECG signals to facilitate cardiac beat detection**
Verfahren und Vorrichtung zur Verbesserung des Signal-Rauschverhältnisses von EKG-Signalen zur Erleichterung der Herzschlagerkennung
Procédé et appareil pour améliorer le rapport signal sur bruit de signaux ECG afin de faciliter la détection du rythme cardiaque

(30) Priority: 30.11.2009 US 264852 P
(43) Date of publication of application: 01.06.2011
(73) Proprietor: Biotronik CRM Patent AG, 6341 Baar (CH)
(72) Inventor: Garner, Garth, Tigard, OR 97224 (US); Lian, Jie, Beaverton, OR 97007 (US); Müssig, Dirk, West Linn, OR 97068 (US)
(74) Representative: Lindner-Vogt, Karin L.

(56) References cited:
- US-A1- 2009 318 821
- WEI D ET AL: "A low-distortion filter method to reject muscle noise in multi-lead electrocardiogram systems", FRONTIERS OF MEDICAL AND BIOLOGICAL ENGINEERING, VSP. ZEIST, NL, vol. 9, no. 4, 1 January 1999 (1999-01-01) , pages 315-330, XP009145991, ISSN: 0921-3775
- KLIGFIELD ET AL: "Recommendations for the standardization and interpretation of the electrocardiogram", HEART RHYTHM, vol. 4, no. 3, 7 March 2007 (2007-03-07), pages 394-412, XP005915945, ISSN: 1547-5271, DOI: DOI:10.1016/J.HRTHM.2007.01.027
- ANONYMOUS: "Electrocardiography I Laboratory", Cleveland Medical Devices, Inc. , 2006, pages 1-17, XP002628270, Retrieved from the Internet: URL:http://biomed.engr.sc.edu/bme_lab/lab% 20reports/36%29%20ECG%20I.pdf [retrieved on 2011-03-15]

## Description

The present invention generally relates to medical devices that measure cardiac electrical signals, analyze the said cardiac electrical signals, and detect the cardiac beat for each cardiac cycle. More particularly, the present invention relates to a method and apparatus for robust cardiac beat detection from surface ECG or subcutaneous ECG signals by improving the signal to noise ratio of ECG signals or subcutaneous ECG signals.

The standard 12-lead electrocardiogram (ECG) is a representation of the heart's electrical activity recorded from electrodes on the body surface. Since its invention by Willem Einthoven in 1904, ECG has been a standard diagnosis tool for evaluating cardiac function. Normal ECG tracing is comprised of different waves that represent the sequence of depolarization and repolarization of the atria and ventricles. For example, the P wave represents atrial depolarization, the QRS complex represents ventricular depolarization, and the T wave represents ventricular repolarization. From these ECG waves, a plural of intervals can be calculated that reflect the cardiac conduction properties (e.g., P wave duration, PR interval, QRS duration), repolarization properties (e.g, QT interval), intrinsic heart rate (e.g., PP or RR intervals), etc. Collectively, these ECG waves and durations contain important diagnostic information regarding the underlying cardiac condition.

However, many patients have intermittent spontaneous cardiac arrhythmias (e.g., sinus bradycardia, non-sustained ventricular tachycardia, paroxysmal atrial fibrillation, etc.) which may not be recorded during their clinic visits. In order to capture these infrequent arrhythmia episodes, external ECG monitoring devices such as Holters are frequently prescribed to continuously monitor the patient's ECG. However, Holter recording has two inherent drawbacks. First, the memory capacity is limited, and most commercially available Holter machines can only record 24-hr or 48-hr surface ECG. Second, the use of skin electrodes is inconvenient and uncomfortable to patient, and is a significant source of measurement noise due to loose contact, muscle movement, and environmental factors. Consequently, the diagnostic yield of Holter ECG is very limited.

To overcome these shortcomings, subcutaneous ECG monitors have been introduced. By implanting a small device with sensing electrodes underneath the skin, the subcutaneous ECG monitor can record subcutaneous ECG which resembles the surface ECG. The subcutaneous ECG monitor can be configured as a loop recorder, so that it continuously records newly acquired subcutaneous ECG while discarding the old recordings. When patient experiences symptoms, he/she can use a handheld device which communicates with the subcutaneous device to trigger a snapshot of the recordings. Alternatively, the implantable device can be programmed to automatically trigger a snapshot of the subcutaneous ECG upon detection of an arrhythmic episode. The recorded snapshots can then be transmitted over the telephone or wireless network to the physician's office for clinical review. Because the loop recorder continuously refresh its memory, it can be carried for long periods, thus is ideal for capturing ECG traces of infrequent episodes such as syncope. Recently, subcutaneous ECG recording has also become a useful means to monitor the cardiac rhythm after ablation of atrial fibrillation, to determine the ablation efficacy and adjust therapeutic schemes.

Irrespective ECG recording apparatus (e.g., ECG machines, bedside ECG monitors, Holter ECG monitors, subcutaneous ECG devices, etc.), reliable beat detection is the prerequisite for further ECG processing and clinical diagnosis. Despite decades of research, ECG beat detection has remained as a technical challenge. On one hand, many factors can cause over-sensing (false detection) of cardiac beats, such as large T waves, wider QRS complexes, muscle noise, electromagnetic interference (EMI), and so on. On the other hand, under-sensing (missed detection) of cardiac beats are also common for ECG signals with small signal to noise ratio. Existing methods for real-time ECG beat detection are either computationally complex that are not suitable for implementation in embedded system, or oversimplified (e.g., rely solely on ECG metrics such as peak amplitude, peak slope, etc., with or without adaptive sensing threshold) that result in unsatisfactory performance.

In view of above, there is a need to provide a method and apparatus for more accurate and efficient detection of cardiac beats based on surface ECG or subcutaneous ECG recordings.

The article by Wei et al., "A low-distortion filter method to reject muscle noise in multi-lead electrocardiogram system", Frontiers of Medical and Biological Engineering, 1999, vol. 9, nr. 4, pages 315 to 330, discloses estimating an arbitrary lead by a linear combination of all other leads, and using this estimated signal to calculate a coherence value c between the actual signal and the corresponding estimated signal. The value of c varies between 0 and 1, and is used to eliminate muscle noise.

The present invention provides a novel method and apparatus for improving the signal to noise ratio of surface ECG or subcutaneous ECG for robust beat detection.

The invention is defined in the appended claims.

According to the present invention, at least three electrodes are connected to a multiplex input that measures surface ECG or subcutaneous ECG in three sensing leads (AB, BC, CA) that form a triangle. The signals measured from two such leads (BC, CA) are combined to calculate their differential signal, which is a first estimate of the signal measured in the 3^{rd} lead. Preferably, the signal acquired by the 3^{rd} lead (AB) is also processed through a moving average filter to produce a second estimate of signal recorded in the same lead. A conditional product signal (P_{AB}) is then obtained by generating the point-by-point product of the differential signal (the first estimate of the signal) and the averaged signal (the second estimate of the signal) if their sample pair have the same polarity, or setting to zero if the sample pair have different polarity. Similarly, conditional product signals (**P**_{BC} and **P**_{CA}) are respectively obtained. These conditional product signals significantly enhance the signal components while reduce the noise components, thus improving the signal to noise ratio of the respective input channels. The conditional product signals (**P**_{AB}, **P**_{BC}, **P**_{CA}) are then respectively subject to peak detection through conventional Auto-Sensing (involve both signal blanking and adaptive thresholding) approach. The preliminary event detection results from all 3 conditional product signals are then aggregated, and the final sense markers for ECG beat detection are generated by means of a voting algorithm.

The details of the invention can be understood from the following drawings and the corresponding text descriptions.
- Fig. 1: shows a block diagram of an implantable subcutaneous ECG monitoring device, and its interfaces with an external programming device and an external portable device, which further communicates with the remote service center.
- Fig. 2: shows a schematic drawing of three spatially distributed electrodes that are capable to measure ECG or subcutaneous signals from three sensing vectors.
- Fig. 3: shows a simplified block diagram of the front-end of the implantable device that includes a multiplexer and a plural of signal pre-processing units.
- Fig. 4: shows a schematic drawing of multiplexing three-channel subcutaneous signals in synchronization with the sampling clock.
- Fig. 5: shows a block diagram of generating three conditional product signals from three multiplexed differential voltage signals.
- Fig. 6: shows a flowchart diagram of implementing the conditional product function.
- Fig. 7: shows a block diagram of generating three channels of phase-corrected conditional product signals and the final sense markers by analyzing three input conditional product signals.
- Fig. 8: shows an example of three channels of time-multiplexed digital subcutaneous ECG signals U_{AB}, U_{BC} and U_{CA}.
- Fig. 9: shows the composite signals Ũ_{AB}, Ũ_{BC}, Ũ_{CA} that are estimated from the time-multiplexed differential signals U_{AB}, U_{BC} and U_{CA}.
- Fig. 10: shows the conditional product signals P_{AB}, P_{BC}, and P_{CA} that are respectively constructed from the first estimated signals Ũ_{AB}, Ũ_{BC}, Ũ_{CA}, and the second estimated signals U̅_{AB}, U̅_{BC} AND U̅_{CA}.
- Fig. 11: shows the phase-corrected conditional product signals S_{AB}, S_{BC}, and S_{CA}.

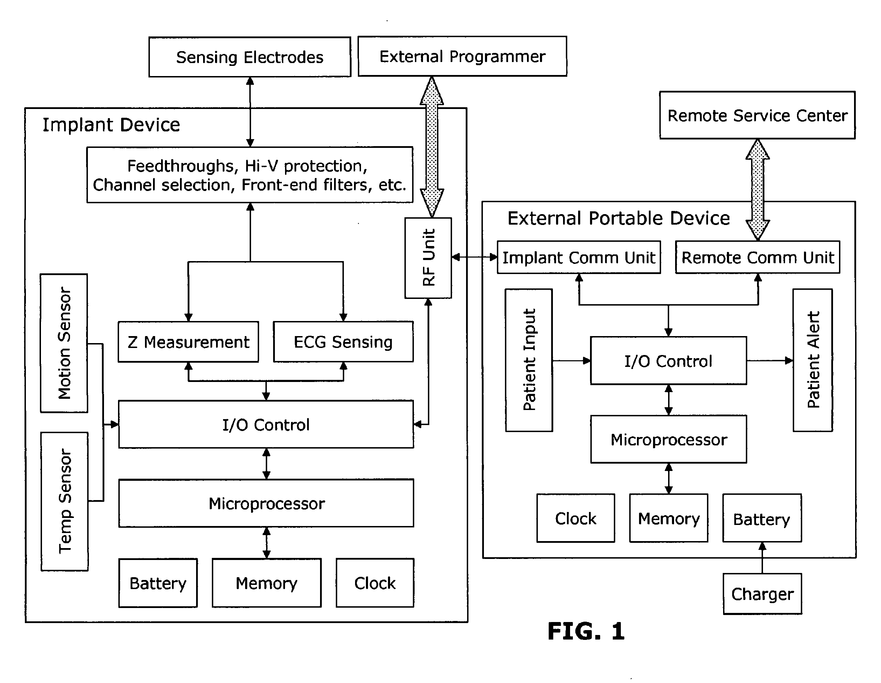

Figure 1 shows a block diagram of an implantable device for subcutaneous ECG monitoring, and its interfaces with an external programmer and an external portable device, which further communicates with the remote service center.

Refer to Figure 1. The implantable device consists of an electronic circuitry that is hermetically sealed inside a Can, which is made from a biocompatible conductive material such as titanium, an optional non-conductive header attached to the Can, three or more sensing electrodes, with or without leads connected to the header.

The sensing electrodes, which are electrically isolated from one another, are mounted over the outer surface of the Can, or outside the header (if available), or at the distal end of the leads (if available). For subcutaneous ECG recording, one or more pairs of sensing electrodes form the sensing vectors and the inter-electrode distance is preferably greater than 3cm.

The leads are optional for subcutaneous ECG recording. Generally, if the measured subcutaneous ECG amplitude is too small for reliable sensing, despite configuring different sensing vectors and recording at different anatomical locations, then one or more subcutaneous leads (with distal electrodes) could be tunneled under the patient's skin and connected to the header, so that larger subcutaneous ECG amplitude could be measured by increasing inter-electrode distance, e.g., between the lead electrode and the Can or header electrode.

Still refer to Figure 1. Enclosed inside the hermetically sealed Can, a microprocessor and associated circuitry make up the controller of the implant device. The implant device is powered by a battery, and maintains an internal clock for timing the operations. The microprocessor communicates with a memory via a bi-directional data bus. The memory typically comprises a ROM or RAM for program storage and a RAM for data storage.

The sensing electrodes are first connected to an electronic interface that preferably includes a feedthrough circuitry for noise reduction, a high voltage protection circuitry, a switch network circuitry for sensing channel selection, and front-end analog filters, as well known in the field. The configurations of the interface circuitry (e.g., filter settings, sensing channel selection, etc.) can be programmed by the microprocessor.

The microprocessor connects to an I/O control unit to manage the input and output of the implant device. One input signal is the subcutaneous ECG picked up by the sensing electrodes. After pre-processed by the interface circuitry, the subcutaneous ECG signal is further processed by the ECG sensing unit, which usually consists of amplifiers, analog-to-digital converters, digital filters, etc., as known in the art.

Another input signal is the impedance (Z) signal measured between the sensing electrodes. By injecting a small constant current (e.g., 100uA, preferably biphasic) between two electrodes while measuring the voltage difference between the same or different pair of electrodes, the impedance is calculated as the ratio between the measured voltage difference and the injecting current strength. As known in the art, the impedance signal provides useful information on the integrity of the sensing channel. In addition, the continuously measured impedance signal may be further processed by the microprocessor to extract other physiological status of the patient, such as the respiration rate.

Other types of biological signals measured by specific sensors can also serve as input to the implant device. For example, an on-board accelerometer can serve as a motion sensor that provides patient's activity signal to the implant device, an on-board (or embedded in the lead) temperature sensor can provide the subcutaneous temperature signal to the implant device. Other types of input signals include, but are not limited to, the subcutaneous pressure signal measured by a pressure sensor, the acoustic signal measured by an acoustic sensor, the subcutaneous pH signal measured by a pH sensor, etc.

By running the program stored in the memory, the microprocessor also sends instructions to the ECG sensing unit, the impedance measurement unit, and other input measurement units to control how these signals are acquired (e.g., gain, offset, filter settings, sampling frequency, sampling resolution, etc.).

The acquired biological signals are then stored in the device memory and analyzed by the microprocessor by running programmed algorithms. For example, the microprocessor continuously analyze the acquired subcutaneous ECG signals to detect the peak of QRS complex. Such QRS peak detection can be achieved by many different means. One typical embodiment is to use an Auto-Sensing algorithm that applies a detection hold-off period after each peak detection, then automatically adjusts the sensing threshold, which is adaptive to the measured peak amplitude of the QRS complex and varies based on a predetermined time dependence. One exemplary Auto-Sensing algorithm has been disclosed in U.S. Pat. No. 5,891,048, assigned to the present assignee. In this invention, a more robust ECG beat detection method is described in details below.

Accordingly, the implant device measures the intervals between any two adjacent peaks of the detected QRS complexes, and these intervals are termed RR intervals. These measured RR intervals are stored in the device memory according to predefined storage modes. One typical mode is the queue-loop mode, meaning the measured RR intervals are stored in a predefined memory space, and while the allocated memory space is full, the newly measured RR intervals replace the oldest stored RR interval data. Another typical mode is the snapshot mode, meaning the measured RR intervals are stored in a predefined memory space, and while the allocated memory space is full, the newly measured RR intervals are not stored until the microprocessor decides to store another episode of RR intervals. Yet another typical mode is the mixed mode, in which one or more segments of allocated memory space store the RR intervals in queue-loop mode, whereas one or more segments of separately allocated memory space store the RR intervals in snapshot mode.

Similarly, the microprocessor can also continuously analyze the acquired subcutaneous ECG signals to measure other metrics of the QRS complex, such as the width of the QRS complex, the positive or negative peak amplitude of the QRS complex, the absolute area under the QRS complex, the maximum positive or negative slopes of the QRS complex, the dominant frequency component of the QRS complex, the complexity measures (e.g., sampled entropy) of the QRS complex, and so on. Likewise, the time series of these measured metrics are stored in the device memory for further analysis.

The implant device also includes a radio-frequency (RF) telemetry unit. The RF telemetry unit may be of the type well known in the art for conveying various information which it obtains from the implant device to the external programmer, or for receiving programming parameters from the external programmer and then conveys to the implant device. In one typical embodiment, the external programmer can interrogate the implant device to get the status of the implant device (e.g., battery status, sensing channel impedance, etc.) or the data recorded by the implant device (e.g., peak amplitude of the QRS complexes, statistics of measured RR intervals, etc.). In another typical embodiment, the external programmer can be used to activate or deactivate selected algorithms or update programmable parameters of the implant device.

In addition, the external portable device to be described hereinafter, can also communicate bi-directionally with the implant device through the telemetry unit. Preferably, the data that may be received from or sent to the external portable device are more limited as compared to the data that may be received from or sent to the external programmer.

In a preferred embodiment, the data that are transmitted from the external portable device to the implant device are simple commands, such as trigger a snapshot of the acquired subcutaneous ECG, retrieve most recently diagnostic information from the implanted device, etc. These commands set the implant device into one of a number of modalities wherein each modality is determined and controlled by parameters that can only be selected by a physician operating the external programmer using secure password or codes.

The data that are transmitted from the implant device to the external portable device preferably include simple acknowledgment to confirm receiving the commands from the external portable device, the signals warning the detection of abnormal conditions, such as detection of atrial fibrillation (AF), detection of high ventricular rate (HVR), detection of low ventricular rate (LVR), detection of abnormal sensing impedance, detection of abnormal temperature, and so on. Other diagnostic information, such as the AF burden, the frequency of ectopic beats, snapshots of RR intervals or subcutaneous ECG, etc., can also be transmitted to the external portable device. Preferably, a physician operating the external programmer using secure password or codes controls the enable or disable condition as well as the amount of data that can be transmitted from the implant device to the external portable device.

Still refer to Figure 1. The external portable device has a power source, such as a lithium battery, which provides power to the electrical components of the device. The battery is chargeable by connecting to an external charger. The external portable device also maintains an internal clock for timing its operations. The overall functioning of the external portable device is controlled by its microprocessor, which reads and performs instructions stored in its associated memory. The instructions stored in memory preferably include instructions defining a communication protocol compatible with the implant device, and instructions defining a communication protocol compatible with the remote service center.

The microprocessor of the external portal device communicates with an I/O control unit to read from the keypad (or press switches) the patient input commands. In an exemplary embodiment, one subset of the input commands is designed to configure the external portable device, for example, to turn on or off certain outputs as described hereinafter, or select specific communication protocols. Another subset of the input commands is designed to establish communication between the external portable device and the remote service center through remote communication unit. For example, patient's input can command the external portable device to transmit diagnostic information (retrieved from the implant device) to the remote service center, and wait to receive acknowledgement. The third subset of the commands is designed to establish communication between the external portable device and the implant device through implant communication unit. For example, patient's input can command the external portable device to transmit corresponding signals to the implant device to trigger recording a snapshot of the subcutaneous ECG, to retrieve diagnostic information from the implanted device, etc. The implant communication unit also receives the acknowledgement and related diagnostic information sent from the implant device, and conveys these data to the microprocessor for storage in the memory.

According to one exemplary embodiment, upon receiving a predefined warning signal from the implant device (e.g., detection of AF, detection of HVR, detection of LVR, detection of abnormal sensing impedance, detection of abnormal temperature, etc.), the microprocessor of the external portable device communicates with the I/O control unit to generate output that is perceptible by the patient. Such output can be in the form of visible message, such as the light-up or blinking of a light emitting diode (LED) or the text message displayed in a liquid crystal display (LCD), or in the form of audible message such as beep, ringing tone, or pre-recorded voice messages played by a speaker, or in the form of discernible vibration by a vibrator. According to the patient's preference, one or multiple types of warning message can be respectively turned on or off. For example, the visible warning message can be turned on while the audible warning message can be turned off during the night if the patient chooses not to be disturbed during sleep even if the implant device detects AF. Besides generating warning messages, some diagnostic information that is received from the implant device and stored in memory (e.g., the heart rate) can also be provided to the patient in the form of visual or audible messages.

The external portable device, via its remote communication unit, can further communicate with the remote service center. Such long-range communication apparatus can be in the form of a mobile radio network, or a fixed-line telecommunication network, or the internet, as well known in the art. Examples of such long-range communication apparatus have been taught in U.S. Pat. No. 6,470,215, U.S. Pat. No. 6,574,509, U.S. Pat. No. 6,622,043.

In one typical embodiment, the external portable device transmits the implant device status information (e.g., battery status, sensing impedance, etc.) as well as relevant diagnostic information (e.g., AF burden, ectopic beat frequency, etc.) to the remote service center according to a predefined transmission frequency and schedule (e.g., every midnight, etc.). Yet in another typical embodiment, the external portable device communicates with the remote service center in a trigger mode, for example, upon receiving a warning signal from the implant device, or upon the patient trigger. In such cases, the external portable device transmits critical diagnostic information stored in device memory (e.g., AF burden, mean heart rate, the subcutaneous ECG snapshot, etc.) to the remote service center.

The remote service center receives the information via compatible communication protocols, then sends acknowledgement back to the external portable device, which may generate visible or audible output indicating receipt of the acknowledgement. The data received by the remote service center is stored in a central database, and is promptly presented to the patient's physician or responsible expert through proper means, such as fax, email, and text message as known in the art. By reviewing the received diagnostic information, the physician can evaluate the patient's condition and provide expert advice to the patient who wishes to contact the physician before taking any action in response to the warning signals generated by the external portable device.

The method and apparatus for cardiac beat detection from subcutaneous ECG recordings is disclosed hereinafter. It shall be understood that the same principles are also applicable to cardiac beat detection from surface ECG recorded using conventional ECG machines, bedside ECG monitors, Holter ECG devices, automatic external defibrillators, etc.

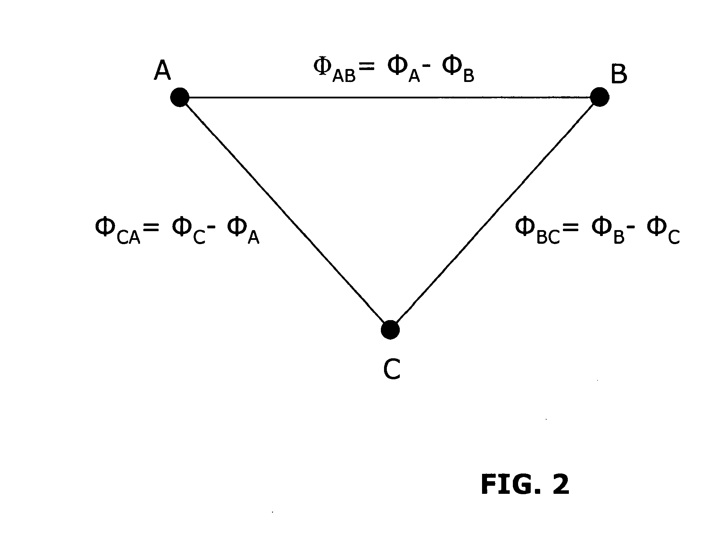

According to this invention, at least three sensing electrodes are connected to the input channels of the implantable device to measure the subcutaneous ECG. The three sensing electrodes are implanted underneath the chest skin and are spatially separated, preferably with inter-electrode distance >3 cm for each pair. As illustrated in Figure 2, three sensing electrodes (labeled as A, B, and C) respectively measure the focal subcutaneous electric potentials Φ_{A}, Φ_{B}, and Φ_{C}. These three sensing electrodes form three sensing vectors or leads that form a triangle. Specifically, electrodes A and B form lead AB that measures Φ_{AB}, which is the voltage difference between Φ_{A} and Φ_{B}. Similarly, electrodes B and C form lead BC that measures Φ_{BC}, which is the voltage difference between Φ_{B} and Φ_{C}. Likewise, electrodes C and A form lead CA that measures Φ_{CA}, which is the voltage difference between Φ_{C} and Φ_{A}. Hence, the triangular voltage measurement provides at least three channels of subcutaneous ECG signal input to the subcutaneous implantable device.

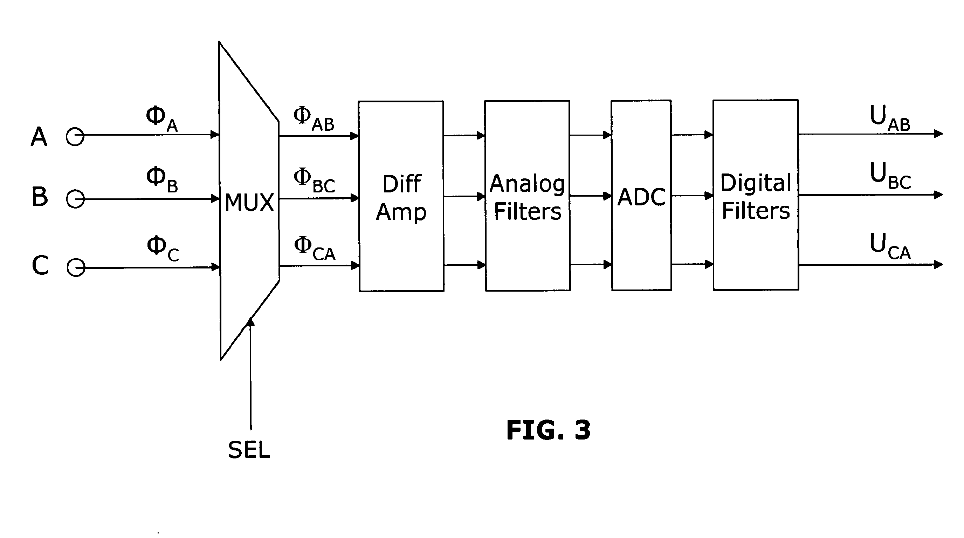

Figure 3 shows a simplified block diagram of the front-end of the implantable device that receives the analog voltage signals from three sensing electrodes and generates three channels of multiplexed digital output. In this figure, the front-end circuit consists of 5 signal processing layers: (1) the multiplexer, (2) the differential amplifiers, (3) the analog filters, (4) the analog-to-digital converter (ADC), and (5) the digital filters.

As shown in Figure 3, three subcutaneous sensing electrodes (A, B, C) respectively measure the focal subcutaneous electric potentials Φ_{A}, Φ_{B}, and Φ_{C}, and are connected to the input of a multiplexer. The multiplexer is controlled by the microprocessor of the implantable device, which divides the time domain into three recurrent timeslots of fixed length, and then sequentially selects two sensing electrodes at a time to obtain three time-multiplexed differential voltage signals Φ_{AB}, Φ_{BC}, Φ_{CA}. In synchronization with the system clock, the microprocessor selects the pair of sensing electrodes according to the sampling frequency which determines the multiplexing timeslot length. For example, if the sampling frequency is 256 Hz, then the multiplexing timeslot length is about 3.9 ms.

Still refer to Figure 3. The multiplexed signals (Φ_{AB}, Φ_{BC}, Φ_{A}) are connected to the differential amplifiers which amplify the differential voltage signals while providing common mode rejection. The amplified differential voltage signals are then connected to the analog filters, which include at least anti-aliasing low pass filters whose cut-off frequency corresponds to the highest frequency component of interest in subcutaneous ECG signals, e.g., 128 Hz. In addition, the analog filters also include high pass filters with sufficiently low corner frequency, e.g., 0.5 Hz, to remove the DC offset component. Preferably, the analog filters also include the 50 Hz or 60 Hz notch filters that remove the power-line interference.

Through the ADC, the amplified and analog-filtered differential voltage signals are then converted to digital signals according to predefined or user-programmable sampling frequency, e.g., 128 Hz, 256 Hz, 512 Hz, etc. The converted digital signals are then further processed through digital filters whose filter characteristics (e.g., filter type, order, gain, corner frequencies, etc.) are user-programmable. By adjusting the digital filter characteristics, user can optimize signal quality for specific applications. For example, for reliable beat detection, T wave can be attenuated to avoid T wave over-sensing.

Therefore, the front-end of the implantable device converts the analog voltage input from three sensing electrodes (Φ_{A}, ΦB and Φ_{C}) to three channels of time-multiplexed digital subcutaneous ECG signals U_{AB}, U_{Bc} and U_{CA}. In a preferred embodiment, the gains and filter settings for all three channels in the front-end are the same.

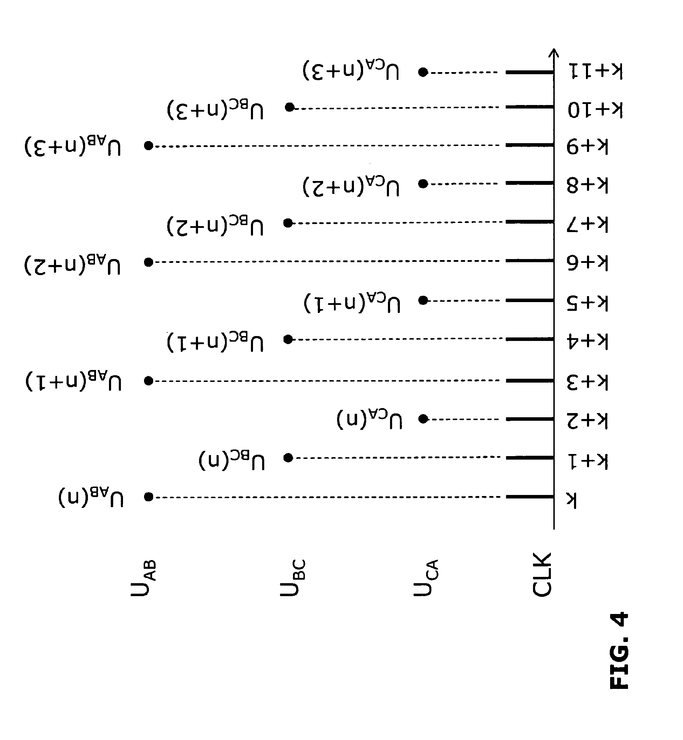

Figure 4 shows a schematic drawing of multiplexing three-channel subcutaneous signals in synchronization with the sampling clock. At the time slot k, the device generates one sample digital output U_{AB}(n) representing the differential voltage in lead AB measured at the time slot k, where n is the sample index. At the next time slot k+1, the device generates one sample digital output U_{BC}(n) representing the differential voltage in lead BC measured at the time slot k+1, where the sample index n remains unchanged. Then at the next time slot k+2, the device generates one sample digital output U_{CA}(n) representing the differential voltage in lead CA measured at the time slot k+2, where the sample index n is still unchanged. Then at the next time slot k+3, the device generates one sample digital output U_{AB}(n+1) representing the differential voltage in lead AB measured at the time slot k+4, where the sample index is increased to n+1. Following the similar steps, the device sequentially generates the sample output U_{BC}(n+1), U_{CA}(n+1), U_{AB}(n+2), U_{BC}(n+2), U_{CA}(n+2), U_{AB}(n+3), U_{BC}(n+3), U_{CA}(n+3) in the following time slots, and the process repeats. As a result, for each specific sample index, there is one digital sample in each of the three output channels. However, these three digital samples represent the differential voltages in respective leads that are measured at three different time slots.

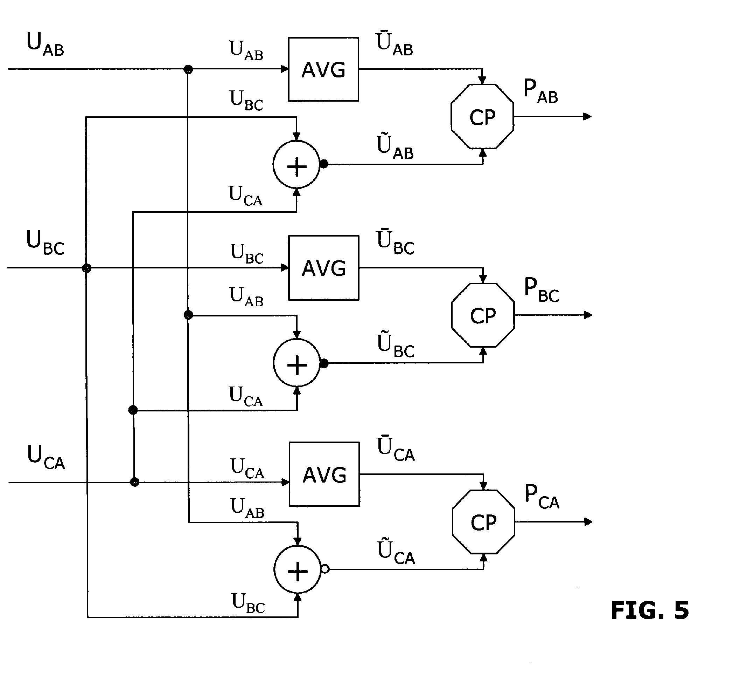

Figure 5 shows a block diagram of generating three conditional product signals (P_{AB}, P_{BC}, P_{CA}) from three multiplexed differential voltage signals (U_{AB}, U_{BC}, U_{CA}).

At any time instant, the cardiac electrical activity can be represented as a 3D dipolar vector, which is projected onto the 3 sensing channels. Therefore, Φ_{BC} measures the far-field projection of the cardiac vector along the BC axis, Φ_{CA} measures the far-field projection of the cardiac vector along the CA axis, and Φ_{AB} measures the far-field projection of the cardiac vector along the AB axis. According to the principles of vector arithmetic, at any time instant t, Φ_{AB}(t)=-(Φ_{BC}(t)+Φ_{CA}(t)), Φ_{BC}(t)=Φ_{AB}(t)+Φ_{CA}(t), Φ_{CA}(t)=-(Φ_{AB}(t)+Φ_{BC}(t)). Because the gains and filter settings for all three channels in the front-end are the same, the same arithmetic relationship would also hold true for the three digital signals had the data been sampled at the same time, that is, at any time instant t, U_{AB}(t)=-(U_{BC}(t)+U_{CA}(t)), U_{BC}(t)=U_{AB}(t)+U_{CA}(t), U_{CA}(t)=-(U_{AB}(t)+U_{BC}(t)).

However, because U_{AB}, U_{BC}, and U_{CA} are time-multiplexed, for each specific sample index n, U_{AB}(n), U_{BC}(n), and U_{CA}(n) are sampled at three adjacent, rather than the same, time slots. Therefore, -(U_{BC}(n)+U_{CA}(n)) is only an approximate of U_{AB}(n), U_{AB}(n)+U_{CA}(n) is only an approximate of U_{BC}(n), and -(U_{AB}(n)+U_{BC}(n)) is only an approximate of U_{CA}(n).

Now refer to Figure 5. The differential voltage signals U_{BC} and U_{CA} are summed and then inversed to generate the signal Ũ_{AB}, which is a first estimate of the signal U_{AB}. Similarly, U_{AB} and U_{CA} are summed and then inversed to generate the signal Ũ_{BC}, which is a first estimate of the signal U_{BC}. Likewise, U_{AB} and U_{BC} are summed and then inversed to generate the signal Ũ_{CA}, which provides a first estimate of the signal U_{CA}.

According to one embodiment, which does not form part of the invention, the signals U_{AB} and Ũ_{AB} are processed through a conditional product (CP) unit to generate the composite signal P_{AB}. Similarly, U_{BC} and Ũ_{BC} are processed through a second CP unit to generate the composite signal P_{BC}, and U_{CA} and Ũ_{CA} are processed through a third CP unit to generate the composite signal **P**_{CA}.

According to the invention, the signals U_{AB} are further processed through a moving average filter to generate the signal U̅_{AB}, which is a second estimate of the signal U_{AB}. Similarly, U_{BC} is further processed through a moving average filter to generate the signal̅ U̅_{BC}, which is a second estimate of the signal U_{BC}, and U_{CA} is further processed through a moving average filter to generate the signal U̅_{CA}, which is a second estimate of the signal U_{CA}. As shown in Figure 5, the conditional product signal **P**_{AB} is then obtained from Ũ_{AB} and U̅_{AB}, which are two different estimates of U_{AB}. Likewise, the conditional product signal P_{BC} is obtained from Ũ_{BC} and U̅_{Bc}, which are two different estimates of U_{BC}, and the conditional product signal P_{CA} is obtained from Ũ_{CA} and U̅_{CA}, which are two different estimates of U_{CA}.

The benefit of using moving average filters is to better align the second estimated signals to the first estimated signals. Refer back to Figure 4. U_{BC}(n) is sampled at the time slot k+1, and U_{CA}(n) is sampled at the time slot k+2. Their combination -(U_{BC}(n)+U_{CA}(n)) yields a first estimate of U_{AB} had it been sampled between the time slots k+1 and k+2. Meanwhile, the average of U_{AB}(n) and U_{AB}(n+1) provides a second estimate of U_{AB} had it been sampled between the same time slots k+1 and k+2. Similarly, U_{AB}(n+1) is sampled at the time slot k+3, and U_{BC}(n+1) is sampled at the time slot k+4. Their combination - (U_{AB}(n+1)+U_{BC}(n+1)) yields a first estimate of U_{CA} had it been sampled between the time slots k+3 and k+4. Meanwhile, the average of U_{CA}(n) and U_{CA}(n+1) provides a second estimate of U_{CA} had it been sampled between the same time slots k+3 and k+4. On the other hand, note that U_{AB}(n+1) is sampled at the time slot k+3, and U_{CA}(n+1) is sampled at the time slot k+5. Their combination U_{AB}(n+1)+U_{CA}(n+1) yields a first estimate of U_{BC} sample at the time slots k+4, which is exactly U_{BC}(n+1). In this case, it is not necessary to apply moving average to obtain a second estimate of U_{BC} because U_{BC} is already aligned with U_{AB}+U_{CA}. Alternatively, we can still apply a moving average filter to generate a second estimate of U_{BC} without changing its alignment with U_{AB}+U_{CA}. For example, U_{BC}(n+1) can be estimated as the average of its two neighboring samples, i.e., (U_{BC}(n)+U_{BC}(n+2))/2, or the weighted average of three adjacent samples, i.e., (U_{BC}(n)+2U_{BC}(n+1)+U_{Bc}(n+2))/4, etc.

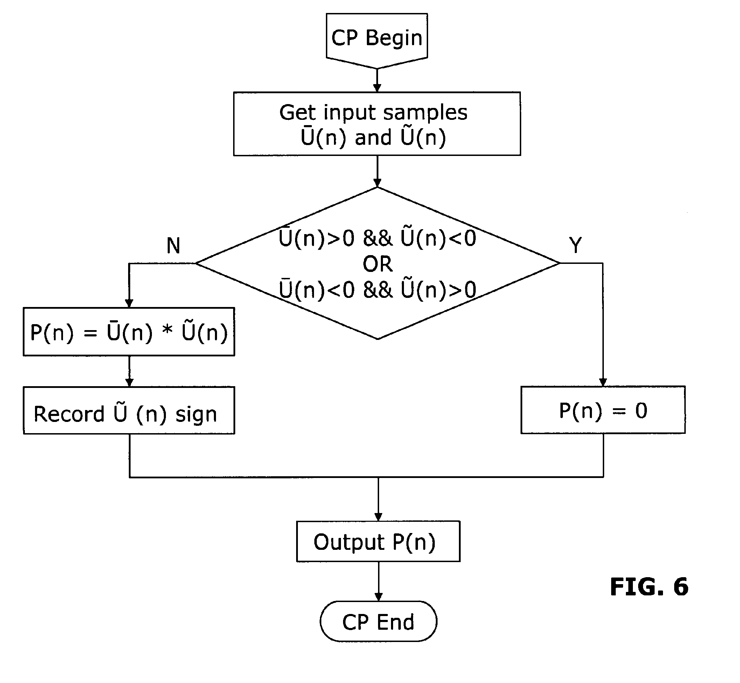

Now refer to Figure 6, which shows a flowchart diagram of implementing the conditional product function. The CP unit continuously process two input signals, Ũ and U̅, to generate the output signal P. For each sample index n, the CP unit gets the pair of input samples U(n) and U(n). If the sample pair have opposite signs, i.e., if Ũ(n)>0 and U̅(n)<0, or Ũ(n)<0 and U̅(n>0, then P(n) is set to 0. Otherwise, P(n) is set to their product, i.e., P(n)=Ũ(n)*U̅̅̅(n). In addition, the sign of U̅(n) or U(n) is recorded for later phase correction as described herein below. The P(n) is the CP output corresponding to the sample index n. Although not shown in this figure, an alternative method is to set P(n)=0 if Ũ(n) and U̅(n) have opposite signs, and set P(n)=Ũ(n)+U̅(n) or P(n)=[Ũ(n)+U̅̅̅(n)]/2 if Ũ(n) and U̅(n) have the same sign.

Normal subcutaneous ECG signal generally has distinctive signal components such as P wave, QRS complex, and T wave. With sufficiently high sampling frequency, each of these signal components can be represented by multiple samples in the digitized signal. For example, a QRS complex with 100 ms duration can be represented by 25-26 samples at the sampling frequency 256 Hz. Consequently, the morphology of the signal component is rarely affected by the input multiplexing. For example, the QRS complex morphology in channel U_{AB} is almost the same no matter the signal is sampled at the time slots k, k+3, k+6, k+9, etc, or sampled at the time slots k+1, k+4, k+7, k+10, etc, or sampled at the time slots k+2, k+5, k+8, k+11, etc.

In contrast, many noises such as myopotentials, EMI, and many other electrical activities originating from outside the heart, have much higher frequency components. These noise components are usually added to the subcutaneous ECG and shown as random impulses with relatively short durations. As a result, these noise components are more sensitive to input multiplexing. For example, assume the sampling frequency is 256 Hz, if a noise impulse has <4 ms duration, then it is very likely that the noise impulse is sampled at one channel at certain time slot, but not sampled at other two channels due to multiplexing. Similarly, at the sampling frequency 256 Hz, a noise impulse with <8 ms duration may be sampled by two channels at two adjacent time slots, but not detected in the third channel. Even if the noise duration is longer and the noise component is sampled in all three channels, the noise component sampled in one channel may have very different morphology than the one estimated from the other two channels, because the noise amplitude may vary significantly from one time slot to the next time slot.

Still refer to Figure 6. Because the signal component is not sensitive to multiplexing whereas the noise component is more sensitive to multiplexing, the CP unit can augment the signal component while suppressing the noise component. For example, assume the subcutaneous ECG in channel U shows a QRS complex at the sample index n. Then the signal component (sample amplitude of QRS waveform) is likely present in both its first estimate Ũ(n) and its second estimate U̅(n), and these two estimates likely have similar sample amplitude with the same sign. Thus, their product P(n)=Ũ(n)*U̅(n) is positive, and augments more for larger sample amplitude (e.g., QRS peak). In contrast, assume the subcutaneous ECG in channel U is contaminated by noise at the sample index n. Then it is very likely that the noise component is not consistently present in both Ũ(n) and U̅(n). For example, noise component may be absent in Ũ(n) and/or U̅(n), or has reduced amplitude in U(n) and/or U(n). Even if noise component is present in both U(n) and U(n), it is likely that U(n) and U(n) have very different amplitude and possibly have different signs. If Ũ(n) and U(n) have different signs, then their conditional product P(n) is set to zero. If Ũ(n) and U(n) have very different amplitude, then P(n) may also be attenuated, e.g., when either U(n) or U(n) is close to zero.

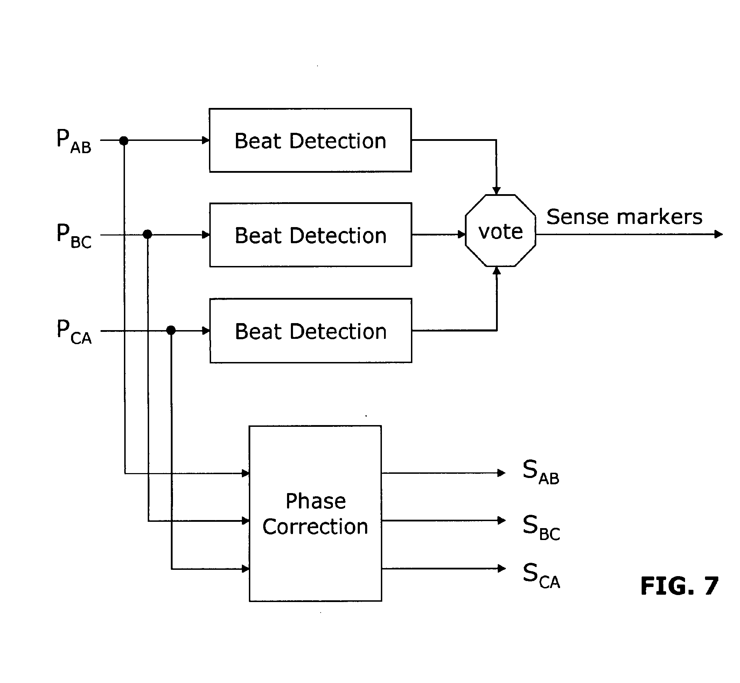

Fig. 7 shows a block diagram of generating three channels of phase-corrected conditional product signals (S_{AB}, S_{BC}, and S_{CA}) and the final sense markers by analyzing three input conditional product signals. Because the conditional product signals P_{AB}, P_{BC}, and P_{CA} are always positive, it is desirable to restore the phase information (positive and negative deflections) embedded in the original signals U_{AB}, U_{BC}, and U_{CA}, for example, to facilitate morphological analysis of the subcutaneous ECG. This is achieved by feeding the conditional product signals P_{AB}, P_{BC}, and P_{CA} to a phase correction unit, which generates respective phase-corrected signals S_{AB}, S_{BC}, and S_{CA}. According to a preferred embodiment, the phase-correction unit retrieves the previously stored sign values of U̅_{AB}(n) or Ũ_{AB}(n) (see Figure 6), and applies the signs to the respective input samples P_{AB}(n) to generate output samples S_{AB}(n). The output samples S_{BC}(n) and S_{CA}(n) can be similarly obtained. According to another embodiment, the phase-corrected conditional product signals (S_{AB}, S_{BC}, and S_{CA}) are obtained by applying previously stored sign values to the square root of the respective conditional product signals P_{AB}, P_{BC}, and P_{CA} so that the output signals have the voltage unit. Yet according to another embodiment (not shown in the figure), the phase-corrected conditional product signal sample S_{AB}(n) is obtained by averaging the first estimate Ũ_{AB}(n) and the second estimate U̅_{AB}(n) but with the exception that S_{AB}(n) is set to zero if Ũ_{AB}(n) and U̅_{AB}(n) have opposite signs.

Still refer to Figure 7. As described above, by amplifying the signal component while suppressing the noise component, the conditional product signals P_{AB}, P_{BC}, and P_{CA} have much higher signal-to-noise ratio (SNR) than their counterparts U_{AB}, U_{BC}, and U_{CA}. According to this invention, the beat detection algorithm is respectively applied to three conditional product signals P_{AB}, P_{BC}, and P_{CA}. One exemplary beat detection algorithm is the Auto-Sensing algorithm, which has been disclosed in U.S. Pat. No. 5,891,048. In brief, the Auto-Sensing algorithm applies a detection hold-off period after each peak detection, then automatically adjusts the sensing threshold, which is adaptive to the measured peak amplitude of the QRS complex and varies based on a predetermined time dependence. Therefore, for each conditional product signal, the beat detection algorithm generates a series of sense markers for that specific channel. According to a preferred embodiment, the resulting three series of sense markers are provided to a vote unit for generating the final sense marker output. Specifically, if a QRS complex is detected in 2 out of 3 conditional product signals within a predefined time window (i.e. tolerance of difference in detection time), e.g. 50 ms, then a sense marker is generated for the final beat detection output. Otherwise (e.g., only one of the three conditional product signals has a beat detection), then no sense marker is generated for the final beat detection output. According to another embodiment of the invention, if and only if a QRS complex is detected in all 3 conditional product signals within a predefined time window (i.e. tolerance of difference in detection time), e.g. 50 ms, then a sense marker is generated for the final beat detection output. Yet according to a further embodiment of the invention, if a QRS complex is detected in any one of the 3 conditional product signals, then a sense marker is generated for the final beat detection output. Other more advanced methods to generate the final beat detection output based on 3 channels of sense markers can also be implemented, for example, by assigning different voting weights to different sense marker channels based on assessment of the signal to noise ratio of P_{AB}, P_{BC}, and P_{CA}, where the signal power can be estimated from the conditional product amplitude at or around the sense marker (i.e. within the QRS complex), and the noise power can be estimated from the conditional product amplitude at or around the middle between adjacent sense markers (i.e. within the T-P segment).

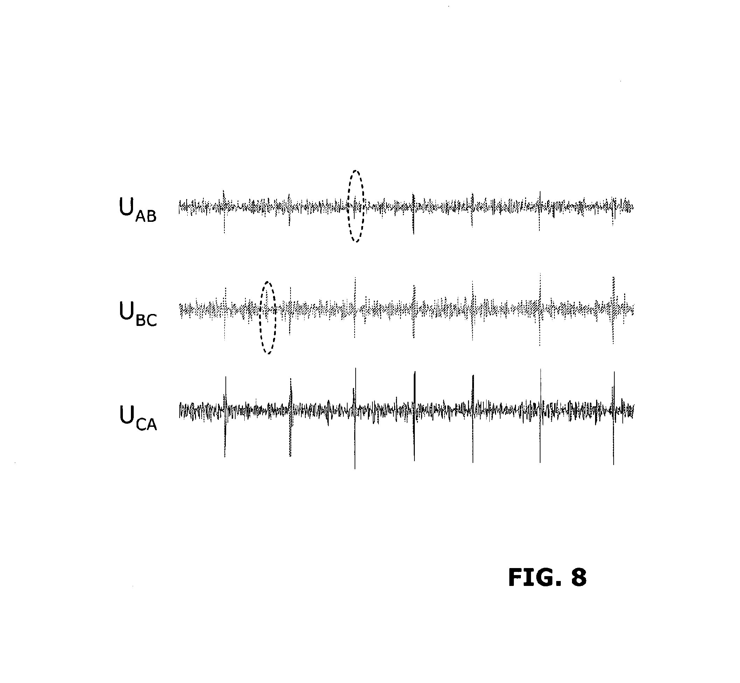

Figure 8 shows an example of three channels of time-multiplexed digital subcutaneous ECG signals U_{AB}, U_{Bc} and U_{CA}. Despite the filtering by the front-end circuit, the signals are contaminated by high frequency noise. The noise power is only slightly lower than the signal power, such that it is difficult to differentiate some true QRS complexes from noise deflections (see for example the marked dashed circles).

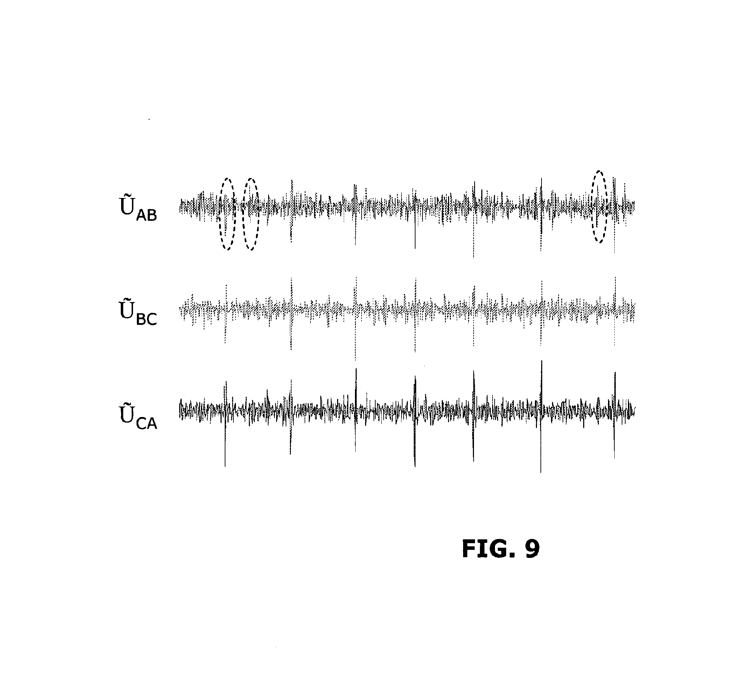

Figure 9 shows the composite signals Ũ_{AB}, Ũ_{BC}, Ũ_{CA} that are estimated from the time-multiplexed differential signals U_{AB}, U_{BC} and U_{CA}. Although in general the estimated signals Ũ_{AB}, Ũ_{BC}, Ũ_{CA} bear some similarity to the respective signals U_{AB}, U_{BC} and U_{CA}, their morphological differences are also evident. Still, the estimated signals are contaminated by high frequency noise. The noise power is only slightly lower than the signal power, such that it is difficult to differentiate some true QRS complexes from noise deflections (see for example the marked dashed circles).

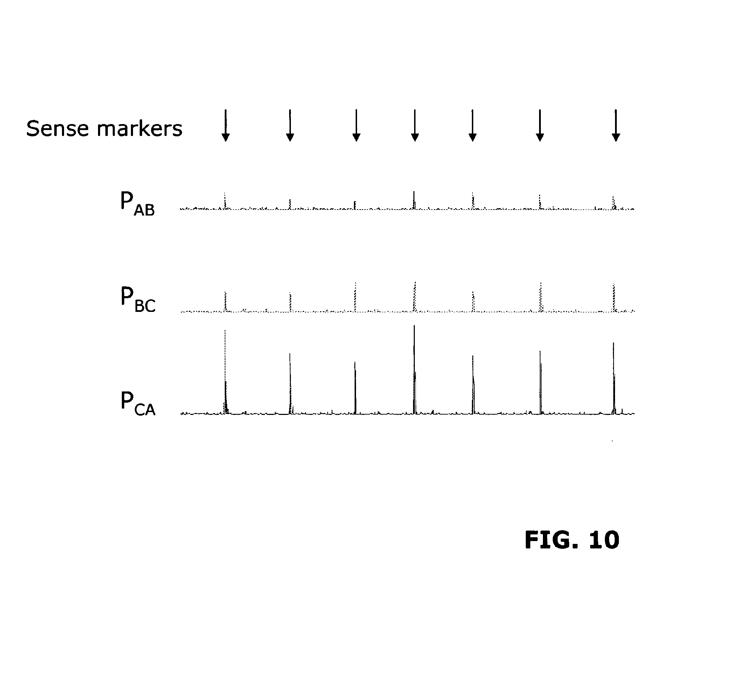

Figure 10 shows the conditional product signals P_{AB}, P_{BC}, and P_{CA} that are respectively constructed from the first estimated signals Ũ_{AB}, Ũ_{BC}, Ũ_{CA}, and the second estimated signals U̅_{AB}, U̅_{BC} and U̅_{CA}. In addition, the final sense markers as determined by the method described above are also shown. Clearly, for each channel, the signal components (QRS complexes) are significantly enhanced whereas the noise components are substantially reduced. The spurious deflections observed in Figure 7 and Figure 8 are either confirmed or rejected as QRS complexes.

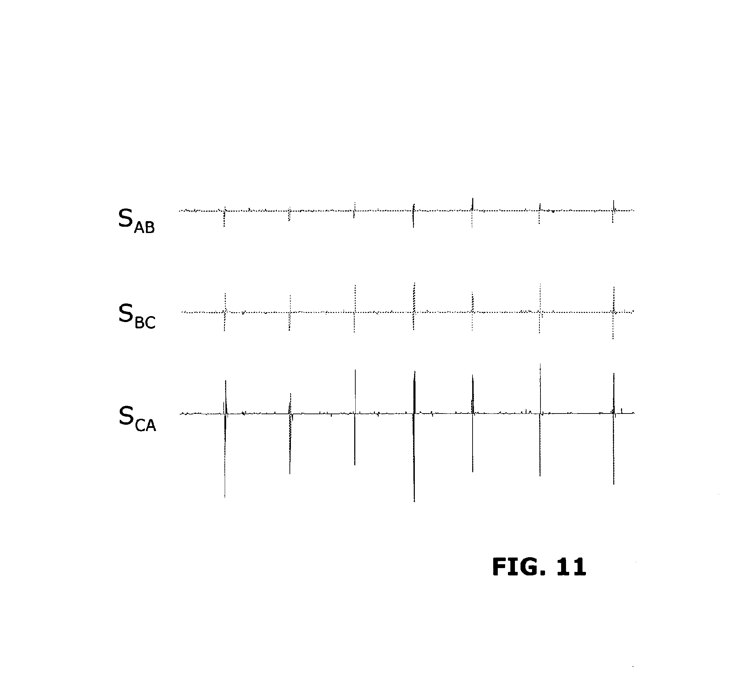

Finally, Figure 11 shows the phase-corrected conditional product signals S_{AB}, S_{BC}, and S_{CA}. Compared to the original subcutaneous ECG signals U_{AB}, U_{BC} and U_{CA}, dramatic improvement of SNR is achieved for S_{AB}, S_{BC}, and S_{CA}. Furthermore, compared to the conditional product signals **P**_{AB}, **P**_{BC}, and **P**_{CA}, the phase-corrected signals S_{AB}, S_{BC}, and S_{CA} also preserve the phase information of the QRS complexes, thus making them more suitable for ECG interpretation and morphological analysis.

## Claims

1. A method of enhancing the signal-to-noise ratio (SNR) of measured electrocardiogram (ECG) signals, the method comprising:
providing at least three cardiac input signals (U_{AB}, U_{BC}, U_{CA}) derived from the measured ECG signals;
forming by means of a microprocessor for each cardiac input signal (U_{AB})
an averaged signal (Ũ_{AB})
an estimated signal (Ũ_{AB}) by combining the other cardiac input signals (U_{BC}, U_{CA}) to calculate their differential signal,
wherein by means of the microprocessor for each cardiac input signal a conditional product (CP) signal is calculated by generating, from each averaged signal and corresponding estimated signal, a product of the averaged and estimated signals if the signals have the same polarity and setting the product to zero if the signals have different polarity.

2. The method of claim 1, **characterized in that** forming an averaged signal is accomplished using a moving average filter.

3. The method of claim 1, **characterized in that** the measured ECG signals are of a type obtained from surface measurements.

4. The method of claim 1, **characterized in that** the measured ECG signals are of a type obtained from subcutaneous measurements.

5. A cardiac device for use in detecting heartbeats, to which at least three sensing electrodes are attached to provide at least three analog voltage input signals, the device comprising:
an electrocardiogram (ECG) sensing unit for use in producing digital signals from the analog voltage input signals comprising a multiplexer and a plurality of signal processing units; and
a programmable microprocessor coupled to the electrocardiogram (ECG) sensing unit;
wherein
the microprocessor is configured to calculate for each of the input signals an averaged signal and an estimated signal by combining the other input Signals to generate their differential signal, and to enhance the SNR of the input signals by generating for each of the input signals a conditional product signal from the averaged signal and corresponding estimated signal if the signals have the same polarity and setting the product signal to zero if the signals have different polarity.

6. The cardiac device of claim 5, **characterized in that** the signal processing units are selected from the group consisting of differential amplifiers, analog filters, analog-to-digital converters, and digital filters.

7. The cardiac device of claim 5 or 6, **characterized in that** the cardiac device is an implantable device for subcutaneous ECG monitoring, further comprising on-board biological input sensors.

8. The cardiac device of any of claim 5 to 7, further comprising an impedance sensor for use in monitoring integrity of the sensing electrodes.

9. The cardiac device of any of claim 5 to 8, **characterized in that** it is implantable in the chest of a patient, the device further comprising an RF unit for use in communicating signals to external parties.

## Patentansprüche

1. Verfahren zur Verbesserung des Signal-Rauschverhältnisses (SRV) von gemessenen Elektrokardiogramm(EKG)-Signalen, wobei das Verfahren Folgendes umfasst:
Bereitstellen von mindestens drei Herzeingangssignalen (U_{AB}, U_{BC}, U_{CA}), die von den gemessenen EKG-Signalen abgeleitet werden;
mittels eines Mikroprozessors für jedes Herzeingangssignal (U_{AB}) Bilden
eines gemittelten Signals (U̅_{AB}) und
eines geschätzten Signals (Ũ_{AB}) durch Kombinieren der anderen Herzeingangssignale (U_{BC}, U_{CA}), um ihr Differenzsignal zu berechnen,
wobei mittels des Mikroprozessors für jedes Herzeingangssignal ein bedingtes Produktsignal (BP-Signal) berechnet wird, indem aus jedem gemittelten Signal und entsprechenden geschätzten Signal ein Produkt aus den gemittelten und
geschätzten Signalen erzeugt wird, wenn die Signale dieselbe Polarität aufweisen und das Produkt auf null gesetzt wird, wenn die Signale unterschiedliche Polarität aufweisen.

2. Verfahren nach Anspruch 1, **dadurch gekennzeichnet, dass** das Bilden eines gemittelten Signals unter Verwendung eines gleitenden Mittelwertfilters erreicht wird.

3. Verfahren nach Anspruch 1, **dadurch gekennzeichnet, dass** die gemessenen EKG-Signale aus Oberflächenmessungen erhalten werden.

4. Verfahren nach Anspruch 1, **dadurch gekennzeichnet, dass** die gemessenen EKG-Signale aus subkutanen Messungen erhalten werden.

5. Herzvorrichtung zur Verwendung bei der Erfassung von Herzschlägen, an der mindestens drei Messelektroden befestigt sind, um mindestens drei analoge Spannungseingangssignale zu liefern, wobei die Vorrichtung Folgendes umfasst:
eine Elektrokardiogramm(EKG)-Erfassungseinheit zur Verwendung bei der Erzeugung digitaler Signale aus den analogen Spannungseingangssignalen, die einen Multiplexer und eine Vielzahl von Signalverarbeitungseinheiten umfasst; und
einen programmierbaren Mikroprozessor, der mit der Elektrokardiogramm(EKG)-Erfassungseinheit gekoppelt ist;
wobei
der Mikroprozessor eingerichtet ist, für jedes der Eingangssignale ein gemitteltes Signal und ein geschätztes Signal zu berechnen, indem die anderen Eingangssignale kombiniert werden, um ihr Differenzsignal zu erzeugen, und das SRV der Eingangssignale zu verbessern, indem für jedes der Eingangssignale ein bedingtes Produktsignal aus dem gemittelten Signal und dem entsprechenden geschätzten Signal erzeugt wird, wenn die Signale dieselbe Polarität aufweisen und das Produkt auf null gesetzt wird, wenn die Signale unterschiedliche Polarität aufweisen.

6. Herzvorrichtung nach Anspruch 5, **dadurch gekennzeichnet, dass** die Signalverarbeitungseinheiten aus der Gruppe ausgewählt sind, die aus Differentialverstärkern, Analogfiltern, Analog-Digital-Wandlern und Digitalfiltern besteht.

7. Herzvorrichtung nach Anspruch 5 oder 6, **dadurch gekennzeichnet, dass** die Herzvorrichtung eine implantierbare Vorrichtung für die subkutane EKG-Überwachung ist, die ferner integrierte biologische Eingangssensoren umfasst.

8. Herzvorrichtung nach einem der Ansprüche 5 bis 7, die ferner einen Impedanzsensor zur Verwendung bei der Überwachung der Intaktheit der Messelektroden umfasst.

9. Herzvorrichtung nach einem der Ansprüche 5 bis 8, **dadurch gekennzeichnet, dass** sie in der Brust eines Patienten implantierbar ist, wobei die Vorrichtung ferner eine HF-Einheit zur Verwendung bei der Übertragung von Signalen an externe Beteiligte umfasst.

## Revendications

1. Procédé pour améliorer le rapport signal sur bruit (SNR) de signaux d'électrocardiogramme (ECG) mesurés, le procédé comprenant :
fournir au moins trois signaux d'entrée cardiaques (U_{AB}, U_{BC}, U_{CA}) issus des signaux d'ECG mesurés ;
former au moyen d'un microprocesseur pour chaque signal d'entrée
cardiaque (U_{AB})
un signal moyenne (U̅_{AB}) et
un signal estimé (Ũ'_{AB}) par combinaison des autres signaux d'entrée cardiaques (U_{BC}, U_{CA}) pour calculer leur signal différentiel,
dans lequel au moyen du microprocesseur pour chaque signal d'entrée cardiaque un signal de produit conditionnel (CP) est calculé par génération, à partir de chaque signal moyenne et signal estimé correspondant, un produit des signaux moyenne et estimé si les signaux ont la même polarité et mettre le produit à zéro si les signaux ont une polarité différente.

2. Procédé selon la revendication 1, **caractérisé par le fait que** former un signal moyenne est accompli à l'aide d'un filtre à moyenne mobile.

3. Procédé selon la revendication 1, **caractérisé par le fait que** les signaux d'ECG mesurés sont d'un type obtenu à partir de mesures de surface.

4. Procédé selon la revendication 1, **caractérisé par le fait que** les signaux d'ECG mesurés sont d'un type obtenu à partir de mesures sous-cutanées.

5. Dispositif cardiaque destiné à être utilisé dans la détection des battements cardiaques, auquel au moins trois électrodes de détection sont attachées pour fournir au moins trois signaux d'entrée de tension analogique, le dispositif comprenant :
une unité de détection d'électrocardiogramme (ECG) destinée à être utilisée dans le production de signaux numériques à partir des signaux d'entrée de tension analogique comprenant un multiplexeur et une pluralité d'unités de traitement de signaux ; et
un microprocesseur programmable couplé à l'unité de détection d'électrocardiogramme (ECG)
dans lequel le microprocesseur est configuré pour calculer pour chacun des signaux d'entrée un signal moyenne et un signal estimé par combinaison des autres signaux d'entrée pour générer leur signal différentiel et pour améliorer le SNR des signaux d'entrée par génération pour chacun des signaux d'entrée d'un signal de produit conditionnel à partir du signal moyenne et du signal estimé correspondant si les signaux ont la même polarité et mettre le signal de produit à zéro si les signaux ont une polarité différente.

6. Dispositif cardiaque selon la revendication 5, **caractérisé par le fait que** les unités de traitement de signaux sont choisis dans le groupe consistant en amplificateurs différentiels, filtres analogiques, convertisseurs analogique-numérique et filtres numériques.

7. Dispositif cardiaque selon l'une des revendications 5 ou 6, **caractérisé par le fait que** le dispositif cardiaque est un dispositif implantable pour la surveillance d'ECG sous-cutanée, comprenant en outre des détecteurs d'entrée biologiques incorporés.

8. Dispositif cardiaque selon l'une quelconques des revendications 5 à 7, comprenant en outre un détecteur d'impédance destiné à être utilisé dans la surveillance de l'intégrité des électrodes de détection.

9. Dispositif cardiaque selon l'une quelconque des revendications 5 à 8, **caractérisé par le fait qu'**il est implantable dans la poitrine d'un patient, le dispositif comprenant en outre une unité RF destinée à être utilisée dans la communication de signaux aux parties externes.
